# EUROPEAN PATENT SPECIFICATION

(11) **EP 3 894 857 B1**
(45) Date of publication and mention of the grant of the patent: **07.09.2022**
(21) Application number: 20719186.7
(22) Date of filing: 15.04.2020
(51) Int. Cl.: G01N 33/543, G01N 33/68, A61K 9/51, A61K 31/00, A61K 38/00, A61K 47/02, B82Y 5/00

(54) **EMPTY POROUS PARTICLES FOR USE IN TREATMENT, PREVENTION AND/OR POSTPONEMENT OF DEGENERATION OF NEURODEGENERATIVE DISEASES, NEURONS AND GLIA**
LEERE PORÖSE PARTIKEL ZUR VERWENDUNG IN DER BEHANDLUNG, PRÄVENTION UND/ODER AUFSCHIEBUNG DER DEGENERATION VON NEURODEGENERATIVEN ERKRANKUNGEN, NEURONEN UND GLIA
PARTICULES POREUSES VIDES DESTINÉES À ÊTRE UTILISÉES DANS LE TRAITEMENT, LA PRÉVENTION ET/OU LE RETARDEMENT DE MALADIES NEURODÉGÉNÉRATIVES ET DE LA DÉGÉNÉRESCENCE DES NEURONES ET DES CELLULES GLIALES

(30) Priority: 15.04.2019 SE 1950470
(43) Date of publication of application: 20.10.2021
(73) Proprietor: Nanologica AB, 151 36 Södertälje (SE)
(72) Inventor: FEILER, Adam, 165 71 Hässelby (SE); KOZLOVA, Elena, Stockholm SE-113 29 (SE); ZHOU, Chunfang, 113 66 Stockkholm (SE); ISRAELSON, Adrian, 85330 Dvira (IL); SHOSHAN-BARMATZ, Varda, 85330 Dvira (IL)
(74) Representative: Swea IP Law AB
(86) International application number: PCT/EP2020/060584
(87) International publication number: WO 2020/212418

(56) References cited:
- WO-A1-2012/004291
- WO-A1-2012/058334
- WO-A1-2020/023417
- ALFONSO E. GARCIA-BENNETT ET AL: "Delivery of Differentiation Factors by Mesoporous Silica Particles Assists Advanced Differentiation of Transplanted Murine Embryonic Stem Cells", STEM CELLS TRANSLATIONAL MEDICINE, vol. 2, no. 11, 2 October 2013 (2013-10-02), pages 906-915, XP055705837, US ISSN: 2157-6564, DOI: 10.5966/sctm.2013-0072
- QI Y ET AL: "Improved selection of LMW over HMW proteins from human plasma by mesoporous silica particles with external modification", TALANTA, ELSEVIER, AMSTERDAM, NL, vol. 80, no. 2, 15 December 2009 (2009-12-15), pages 703-709, XP026693055, ISSN: 0039-9140, DOI: 10.1016/J.TALANTA.2009.07.050 [retrieved on 2009-08-03]

## Description

### Field of the invention

The present invention relates to empty porous particles having a diameter between 0.1 and 1000 µm, as measured by SEM, for use in prevention and/or postponement of neurodegenerative diseases, or for prevention and/or postponement of degeneration of neurons and glia.

### Background of the invention and prior art

Formation of abnormal protein aggregates are key factors in neurodegenerative diseases (NDDs), such as Alzheimer's disease (AD), Parkinson's disease (PD), Huntington's disease, Pick's disease and amyotrophic lateral sclerosis (ALS). These aggregates interfere with cellular function and homeostasis, leading to progressive loss of synapses and neurons. Recent findings indicate that one of the factors underlying disease progression in these conditions is intercellular propagation of mutant/pathogenic proteins, resembling the intercellular transfer of prion proteins. Experimental findings supporting that this mechanism occurs for amyloid-beta and tau in AD (e.g. Goedert et al, Propagation of Tau Aggregates and Neurodegeneration. Annu Rev Neurosci. 2017 Jul 25;40:189-210. doi: 10.1146/annurev-neuro-072116-031153. Review), alpha-synuclein in PD (e.g. Wong YC, Krainc D. α-synuclein toxicity in neurodegeneration: mechanism and therapeutic strategies. Nat Med. 2017 Feb 7;23(2):1-13. doi: 10.1038/nm.4269.Review) and various forms of mutant proteins in ALS (e.g. Fatima et al, Spread of pathology in amyotrophic lateral sclerosis: assessment of phosphorylated TDP-43 along axonal pathways. Acta Neuropathol Commun. 2015 Jul 28;3:47. doi: 10.1186/s40478-015-0226-y) including SOD1. Preventing intercellular transfer of mutant/pathogenic proteins may therefore be an attractive therapeutic strategy to counteract the progression of NDDs. Mesoporous silica particles are attractive as a robust and tunable delivery platform for formulation of therapeutic agents. Their use as drug delivery vehicles has resulted in many advances in areas as diverse as pharmaceutical formulation, controlled drug release and diagnostics. WO2012/004291 discloses that mesoporous silica particles (MSPs), loaded with morphogens and co-transplanted with stem cells, assist in differentiation of mouse embryonic stem cells (ESCs) towards motor neuron (MN) progenitors. Subsequent treatment with peptide mimetics of Glial cell-derived neurotrophic factor (GDNF) and/or Ciliary neurotrophic factor (CNTF) markedly promoted survival and specific differentiation of transplanted mouse ESC-derived MN progenitors to functionally competent MNs. Moreover, mimetics released from this co-transplanted delivery system created a gradient from the site of implantation that stimulated directional growth of axons from the differentiating MNs.

Recent studies demonstrated that mimetic loaded MSP induced regeneration of sensory axons after dorsal root avulsion injury in mice, further supporting their usefulness and biocompatibility in in vivo experimental conditions (Hoeber et al, A Combinatorial Approach to Induce Sensory Axon Regeneration into the Dorsal Root Avulsed Spinal Cord. Stem Cells Dev. 2017 Jul 15;26(14):1065-1077. doi: 10.1089/scd.2017.0019. Epub 2017 May 31).

Nanoparticles have also shown the potential to sequester biomolecules and to immobilize proteins. Nontoxic hydrogel copolymer nanoparticles were shown to efficiently sequester and neutralize venomous phospholipase A2 (O'Brien J, Lee SH, Onogi S, Shea KJ. Engineering the Protein Corona of a Synthetic Polymer Nanoparticle for Broad-Spectrum Sequestration and Neutralization of Venomous Biomacromolecules. J Am Chem Soc. 2016 Dec 28;138(51):16604-16607. doi: 10.1021/jacs.6b10950. Epub 2016 Dec 16).

Nanoparticles, named nanosponges and composed of a polymeric core, coated with red blood cell membrane, were able to efficiently bind pore-forming toxins from several pathogenic bacterial strains (Hu CM, Fang RH, Copp J, Luk BT, Zhang L. A biomimetic nanosponge that absorbs pore-forming toxins. Nat Nanotechnol. 2013 May;8(5):336-40. doi: 10.1038/nnano.2013.54. Epub 2013 Apr 14). Silica nanoparticles readily adsorb proteins and other biomolecules in vivo and have shown high affinity for adsorbing RNA-binding proteins (Klein G, Mathé C, Biola-Clier M, Devineau S, Drouineau E, Hatem E, Marichal L, Alonso B, Gaillard JC, Lagniel G, Armengaud J, Carrière M, Chédin S, Boulard Y, Pin S, Renault JP, Aude JC, Labarre J. RNA-binding proteins are a major target of silica nanoparticles in cell extracts. Nanotoxicology. 2016 Dec;10(10):1555-1564. Epub 2016 Oct 25). Mutant RNA-binding proteins and their aggregates are implicated in ALS pathogenesis (e.g. Fatima et al, Acta Neuropathol Commun. 2015 Jul 28;3:47. doi: 10.1186/s40478-015-0226-y). Mesoporous silica particles (MSP) have shown efficient incorporation of free fatty acids in vitro (Valenstein et al, Functional mesoporous silica nanoparticles for the selective sequestration of free fatty acids from microalgal oil. ACS Appl Mater Interfaces. 2012 Feb;4(2):1003-9. doi: 10.1021/am201647t. Epub 2012 Feb 3). Oral intake of MSP with a specific pore size was shown to induce weight loss and reduce blood lipid levels in rats, possibly as a result of sequestering enzymes/bioactive molecules involved in intestinal lipid absorption (Kupferschmidt et al, Large pore mesoporous silica induced weight loss in obese mice. Nanomedicine (Lond). 2014 Jul;9(9):1353-62. doi: 10.2217/nnm.13.138. Epub 2014 Jan 7).

US2018/0256747 discloses use of particles to bind and inhibit biomolecules for use in prevention and/or treatment of neurodegenerative diseases. The particles have an immobilized agent (e.g. TNF) bound to the inner or outer surface of the particles, whereby the agent selectively binds to a biomolecule.

WO2012/013617 discloses a molecularly imprinted polymer (MIP) having a C- or N- terminal epitope(s) of amyloid peptides or isomers thereof, such that the MIP can selectively bind to soluble fractions of amyloid peptides present in body fluids, such as cerebrospinal fluid.

WO2012/145428 discloses a device, whereby at least one protease is bound to a support of the device. The support may be porous particles.

Garcia-Bennet, et al., Stem Cells Transl Med, 2013, 2(11), p 906-915, and WO2012004291 disclose use of particles loaded with peptide mimetics for use in differentiation of stem cells, such as embryonic stem cells, by co-transplantation of particles with embryonic stem cells (ESCs). The particles may be loaded with Cintrofin and Gliafin. The particles loaded with peptide mimetics and co-transplanted have a positive effect on the volume and neurite growth. The results show that unloaded particles transplanted with ESCs have no effect on differentiation of ESCs. These documents are silent about prevention of neurodegenerative diseases or delaying onset of such diseases using porous particles.

There is an increasing need for prevention and/or postponement of formation and propagation of abnormal protein aggregates in affected nervous system and thus prevention and/or postponement of neurodegenerative diseases. With the general population getting older, the incident of the diseases mentioned above, especially Alzheimer's disease, are expected to increase. Today, more than 30 million people worldwide are affected with NDDs, and there are no cures for these diseases, nor are there satisfying methods available to prevent and/or postpone the onset of these diseases.

### Summary of the invention

The invention is defined in the appended claims.

J It is an object of the present invention to at least partly overcome the above-mentioned problems and to provide an improved product for use in prevention and/or postponement of degeneration of neurons and glia, especially motor neurons and neurodegenerative diseases (NDDs).

This object is achieved by empty porous particles having a diameter between 0.1 and 1000 µm, as measured by e.g. SEM, for use in prevention and/or postponement of neurodegenerative diseases. The object is also achieved by empty porous particles having a diameter between 0.1 and 1000 µm, as measured by e.g. SEM, for use in delaying onset of the neurodegenerative diseases. This object is achieved by empty porous particles having a diameter between 0.1 and 1000 µm, as measured by e.g. SEM, for use in prevention and/or postponement of degeneration of neurons and glia. In one aspect, the use relates to treatment of degeneration of neurons and glia or treatment of neurodegenerative diseases.

In an aspect, the use relates to a delay of the progress of neurodegenerative diseases (NDDs).

In some aspects, the neurodegenerative diseases (NDDs) is selected from the group comprising or consisting of Alzheimer's disease (AD), Parkinson's disease (PD), amyotrophic lateral sclerosis (ALS), Huntington's disease (HD), dementia with Lewy bodies (DLB), progressive supranuclear palsy (PSP), multiple system atrophy (MSA), corticobasal degeneration (CBD), frontotemporal dementia (FTD), spinocerebellar ataxia (SCA) disorders, and spinal muscular atrophy or other NDDs. In one aspect, the NDD is ALS.

In one aspect, co-transplantation of stem cells is disclaimed. In another aspect, the empty particles have no surface bound molecules or agents, such as TNF, protease or amyloid peptides.

The results show that administration by injection of empty porous particles or porous particles containing mimetics significantly delayed the disease course of SOD1 mutant mice.

It has been found that the empty porous particles delay the onset of the disease, such as ALS. The particles reduce the time for loss of muscle power as measured by a grip strength test by 20 days in a murine model. In the same model, it was shown that animal survival was increased by 25% after administration of the empty porous particles alone, and 12% after administration of the particles loaded with peptidomimetics (hereinafter called mimetics).

In one aspect, the empty porous particles as defined herein are for use in increasing survival of an ALS patient by at least 12% or at least 25%.

Some of the diseases as mentioned above, such as AD and HD are characterized by abnormal protein aggregates. Therefore, it is reasonable to assume that the onset or progression of these diseases can be delayed by use of the empty porous silica particles.

Without wishing to be bound by any theories, the effect of the empty porous particles may be caused by an uptake of substances/biomolecules from the spinal fluid and/or from the extracellular compartment in the central nervous system parenchyma by the empty porous particles. The effect of the empty porous particles may also be caused by an uptake/absorption/sequestering of substances/biomolecules from inside the cells. The toxic substances/biomolecules may be molecules that cause the disease or cause progression of the disease in the mammal. Porous particles may sequester bio(active) molecules and immobilize enzymes and proteins. This removal of the bio-molecules may prevent and/or postpone or delay the onset of neurodegenerative diseases, such as AD, ALS, PD, etc..

In one aspect, the empty porous particles having a diameter between 0.1 and 1000 µm, as measured by e.g. SEM, are for use in sequestering SOD1, TDP-43, tau and other biomolecules that affect degeneration of neurons and glia. In one aspect, the use is in sequestering SOD1.

In another aspect, the empty porous particles having a diameter between 0.1 and 1000 µm, as measured by e.g. SEM, are for use in sequestering SOD1, TDP-43, amyloid-beta, alpha-synuclein, tau, ELAVL4, FUS and other biomolecules that contribute to progression of neurodegenerative diseases (NDDs), such as Alzheimer's disease (AD), Parkinson's disease (PD), Huntington's disease, Pick's disease, multiple sclerosis (MS) and amyotrophic lateral sclerosis (ALS), or other NDDs. In one aspect, the use is in sequestering SOD1.

In a further aspect, the empty porous particles having a diameter between 0.1 and 1000 µm, as measured by e.g. SEM, are for use in sequestering SOD1, TDP-43, amyloid-beta, alpha-synuclein, tau, ELAVL4, FUS and other biomolecules that contribute to progression of neurodegenerative diseases (NDDs) before, during or after treatment of a mammal with all or at least a portion of the particles loaded with trophic factors, such as peptide mimetics of Glial cell-derived neurotrophic factor (GDNF) and/or Ciliary neurotrophic factor (CNTF). The factors may be administered together Stem Cells. In one aspect, the use is in sequestering SOD1.

In yet another aspect, the empty porous particles having a diameter between 0.1 and 1000 µm, as measured by e.g. SEM, are for use in sequestering SOD1, TDP-43, amyloid-beta, alpha-synuclein, tau, ELAVL4, FUS and other biomolecules that contribute to progression of neurodegenerative diseases (NDDs) before, during or after treatment of a mammal with Stem Cells. In one aspect, the use is in sequestering SOD1.

In one aspect, the empty porous particles are for use in prevention and/or postponement of NDDs in a mammal, whereby the empty particles are administered before, during or after treatment of the mammal with, all or at least a portion of, the particles loaded with trophic factors selected from peptide mimetics of Glial cell-derived neurotrophic factor (GDNF) and/or Ciliary neurotrophic factor (CNTF) and/or Stem Cells. In one aspect, at least 50w/w% of the particles are loaded. In another aspect, the empty particles are administered before the treatment of the mammal with the loaded or partial loaded particles. In a further aspect, the empty particles are administered by injection in the cerebrospinal fluid (CSF), brain or spinal cord parenchyma.

In another aspect, the empty porous particles are for use in prevention and/or postponement of NDDs in a mammal, whereby the empty particles are administered before, during or after treatment of the mammal with, all or at least a portion of, the particles loaded with trophic factors selected from peptide mimetics of Glial cell-derived neurotrophic factor (GDNF) and/or Ciliary neurotrophic factor (CNTF). In one aspect, at least 50w/w% of the particles are loaded. In another aspect, the empty particles are administered before the treatment of the mammal with the loaded or partially loaded particles. In a further aspect, the empty particles are administered by injection in the cerebrospinal fluid (CSF), brain or spinal cord parenchyma.

In one aspect, the empty particles are nanoporous and/or mesoporous silica particles. In another aspect, the empty particles are mesoporous silica particles. In a further aspect, the empty particles are amorphous silica particles.

In one aspect, the empty particles have a diameter between 0.1 and 500 µm, or between 0.1 and 250 µm, or between 0.1 and 100 µm, or between 0.2 and 50 µm, or between 0.3 and 25 µm, or between 0.3 and 20 µm, or between 0.3 and 12 µm, or between 0.3 and 6 µm. In one aspect, the empty particles have a diameter 0.1 to 0.25 µm. This range is especially suitable for injection by a syringe.

In one aspect, the empty particles have a diameter below 1 mm or below 0.2 µm. The particles may penetrate the cells and the intracellular uptake of the substances or biomolecules that cause or prolong the disease may further improve the efficiency of the empty particles in prevention and/or postponement of neurodegenerative diseases. A combination of empty particles having different sizes may be used in the invention to ensure both extracellular and intracellular uptake/absorption/sequestering of SOD1, TDP-43, amyloid-beta, alpha-synuclein, tau, ELAVL4, FUS and other biomolecules that contribute to progression of neurodegenerative diseases (NDDs).

In another aspect, the empty porous particles have a pore volume between 0.1 and 5 cm³/g, or between 0.2 and 3 cm³/g, as measured by nitrogen adsorption isotherm and calculated using the BET (Brunauer-Emmett-Teller) model to calculate the pore volume.

In a further aspect, the empty porous particles have an average pore size between 1 and 250 nm, or between 2 and 50nm as measured by nitrogen adsorption isotherm and calculated using the BET (Brunauer-Emmett-Teller) model to calculate the pore volume and pore size preferably with 2-30 nm , or 5-25nm.

In another aspect, the empty particles are mesoporous silica particles having a pore size between 1 and 100 nm, a pore volume between 0.1 and 3 cm³/g and a surface area between 40 and 1500 m²/g.

In yet another aspect, the empty particles have a pore size between 0.3 and 20 nm, a pore volume between 0.5 and 1.5 cm³/g and a surface area between 50 and 800 m²/g.

In one aspect, the empty particles are hydrophilic and have a surface chemistry of terminal hydroxyl groups -OH. This surface chemistry allows chemical binding with biomolecules via hydrogen bonding and electrostatic interactions.

In yet another aspect, the empty particles have hydrophobic surface chemistry that promotes adsorption of biomolecules via hydrophobic interactions.

In one aspect, the empty porous particles or porous silica particles are biotinylated. In another aspect the empty porous particles or porous silica particles are chemically modified with chemical functionalities including -COOH, -NH₃, OCH₃, -OCH₂CH₃.

In one aspect, the empty porous particles are chemically modified by having a surface chemistry of terminal hydroxyl groups -OH, or having hydrophobic surface chemistry, or having chemical functionalities including -COOH, -NH₃, OCH₃, -OCH₂CH₃, or mixtures thereof. A mixture of particles having different chemical modification may also be used.

In one aspect, the empty porous particles having a diameter between 0.1 and 1000 µm, as measured by e.g. SEM, and defined anywhere above, are for use in diagnosis of a neurodegenerative diseases (NDDs). Certain compounds, such as proteins, can be identified in the cervical and/or spinal fluid of a mammal being ill with a certain disease. By administering the porous particles, empty or loaded with an incubator fluid, and retrieving a portion of the particles after a period of time, such as 1 to 36 hours, the compounds that have been loaded into the particles during their presence in the cervical and/or spinal fluid, can be identified and thus used to diagnose said certain disease.

Also described herein is a method of identifying biomarkers for diagnosis and/or treatment of NDDs, such as ALS, comprising or consisting the steps of:
a) administering the empty porous particles as defined herein, into the cerebrospinal fluid (CSF);
b) retrieving a portion of said porous particles after a period of time, such as 1 to 36 hours, from the CSF;
c) determining biomolecules that have been loaded into the porous particles during their presence in the CSF;
d) establishing if the compounds can be used as biomarkers.

Administration means in vivo administration as well as in vitro addition of empty particles to CSF retrieved from a mammal. Thus, the method may be performed in vivo and in vitro.

Here described is also a method of identifying biomarkers for diagnosis and/or treatment of NDDs, such as ALS, comprising or consisting the steps of:
a) adding the empty porous particles as defined herein, to the cerebrospinal fluid (CSF) retrieved from a mammal;
b) retrieving a portion of said porous particles after a period of time, such as 1 to 36 hours, from the CSF;
c) determining biomolecules that have been loaded into the porous particles during their presence in the CSF;
d) establishing if the compounds can be used as biomarkers.

The empty particles can thus be used in a simple, effective and efficient method at relatively low cost for diagnosing an NDD. "A portion" means part of the administrated or added particles, such as 99w/w% or less.

The empty particles allow for identification of new biomarkers that may help in diagnosing and treatment of NDDs.

The porous particles may be manufactured according to a number of processes. In an aspect the porous particles are manufactured using a silica source such as a silicate such as tetraethoxysilane TEOS, or alternatively a silicate such as sodium silicate or in combination with a pore directing agent or porogen or templating agent such as a surfactant or polymer. In another aspect, the porous particles are manufactured by a sol-gel method, such as the Stöber process, or by a spray drying method. In a further aspect, particles are manufactured by a sol-gel method comprising a condensation reaction of a silica precursor solution with a non-miscible organic solution, oil, or liquid polymer in which droplets are formed by stirring or spraying the solution followed by gelation of the silica by means of change in pH and/or evaporation of the aqueous phase.

The particles comprise a modified surface. The surface may be a chemically modified surface, such as by etching. The surface may be a coated surface. Coating may be used to adhere a bioactive compound, such as a mimetic, to the surface of the particles. In one example not part of the invention, the particles are coated with a functionalizing agent.

In an aspect, the porous particles are manufactured by a process comprising or consisting of the steps of
- provide the empty porous silica particles,
- optionally loading all or at least a portion of the obtained particles with one or more mimetic, and/or
- optionally loading all or at least a portion of the particles with one or more additional agent. In one aspect, an incubator liquid is loaded in or on all or at least in a portion of the empty particles. The incubator liquid is a buffered solution having a physiological pH suitable for administration by injection. Between 5 to 99 w/w% of the empty particles may be loaded, or between 10 and 90 w/w%, or between 25 and 75 w/w%.

In a further aspect, one or more mimetic is loaded in or on all or at least a portion of the empty particles without fixedly being bound to the surface of the particles. The mimetic may be selected from the group comprising or consisting of Gliafin and Cintrofin. Between 5 to 99 w/w% of the empty particles may be loaded, or between 10 and 90 w/w%, or between 25 and 75 w/w%.

The particles loaded with one or more mimetic are believed to increase neurite outgrowth of motor neurons (MNs). The particles loaded with the mimetic are believed to improve motor neuron density in spinal fluid embryonic stem cells (ESC or mESC) or derived SOD1 MNs or SOD1G93A MNs. The particles loaded with one or more mimetic are believed to improve survival of neurons and glia, or MNs or derived SOD1 MNs or SOD1G93A MNs survival by 100% as compared to control (no treatment).

In one aspect, one or more additional agent is loaded in or on the particles. The one or more agent may be selected from the group comprising or consisting of growth factors, pH regulators, antibiotics, anti-inflammatory drugs and immune-suppressors.

Also described herein is a pharmaceutical composition comprising or consisting of a plurality of empty porous particles as defined above, together with a solvent, such as an injection solution or water, and optionally pharmaceutical acceptable additives, for use in prevention and/or postponement of neurodegenerative diseases, such as Alzheimer's disease (AD), Parkinson's disease (PD), Huntington's disease, Pick's disease, multiple sclerosis (MS) and amyotrophic lateral sclerosis (ALS). In one aspect, the NDD is ALS.

All or at least a portion of the empty particles may be loaded with an incubation liquid, and/or one or more mimetic and/or additional agent. The pH of the composition is between 5 and 8, or 6 and 7, or about 6.8. Between 5 to 99 w/w% of the empty particles may be loaded, or between 10 and 90 w/w%, or between 25 and 75 w/w%.

In one aspect, the pharmaceutical composition comprising or consisting of said loaded porous particles together with a solvent, and optionally pharmaceutical acceptable additives, may be used for extended release of one or more mimetic and/or additional agent.

In another aspect, the empty particles are administered by injection in the cerebrospinal fluid (CSF), brain or spinal cord parenchyma. In one aspect, intravenous administration is disclaimed.

In a further aspect, the pharmaceutical composition is administered by injection in the spinal and/or cervical fluid. In yet a further aspect, the pharmaceutical composition is administered by injection in the spinal fluid. In yet another aspect, the pharmaceutical composition is administered by injection in the cervical fluid.

Also described herein is a dosage regime for administration of the pharmaceutical composition as defined above, wherein the pharmaceutical composition is administered by injection into the cerebrospinal fluid or into the brain or spinal cord parenchyma at least once a month. In one aspect, the pharmaceutical composition, as defined above, is administered by injection intrathecally into the spinal cord parenchyma between the third and fourth vertebrae to prevent or postpone degeneration of MNs innervating diaphragm and therefore prevent or postpone degeneration of MNs responsible for breathing.

In one aspect, the pharmaceutical composition, as defined above, is administered by injection in the cerebrospinal fluid, brain or spinal cord parenchyma at least once in one, two, three, or four months.

The invention also related to a use of the empty particles as defined anywhere above in a dosage regime, whereby the particles are administered at least once every one, two, three, or four months.

### Brief description of the drawings

The invention will now be explained more closely by the description of different embodiments of the invention and with reference to the appended figures.

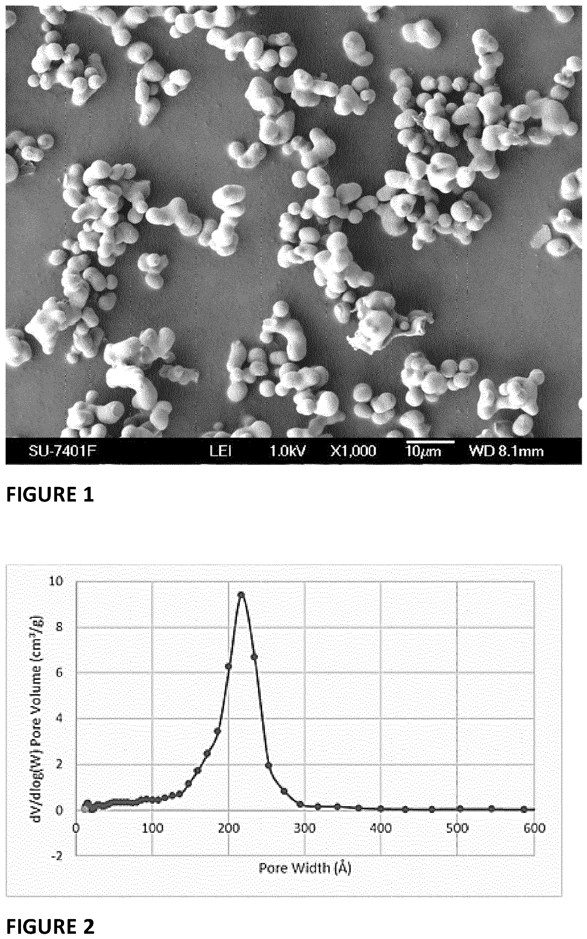

Fig. 1 shows Scanning Electron Microscopy (SEM) of empty mesoporous silica particles used for the sequestering of biomolecules and loading mimetics. The particles consist of aggregates in the size range of 1-10 microns made up from several primary particles of the size of 500nm
Fig. 2 shows pore size distribution of empty mesoporous silica particles obtained by Nitrogen (N₂) Adsorption-Desorption technique using Micromeritics's TriStar II 3020. The pore size was determined to be 23 nm using a Density Functional Theory (DFT) model. The Brunauer-Emmett-Teller (BET) surface area of nanoporous silica was determined to be 722 m²/g.

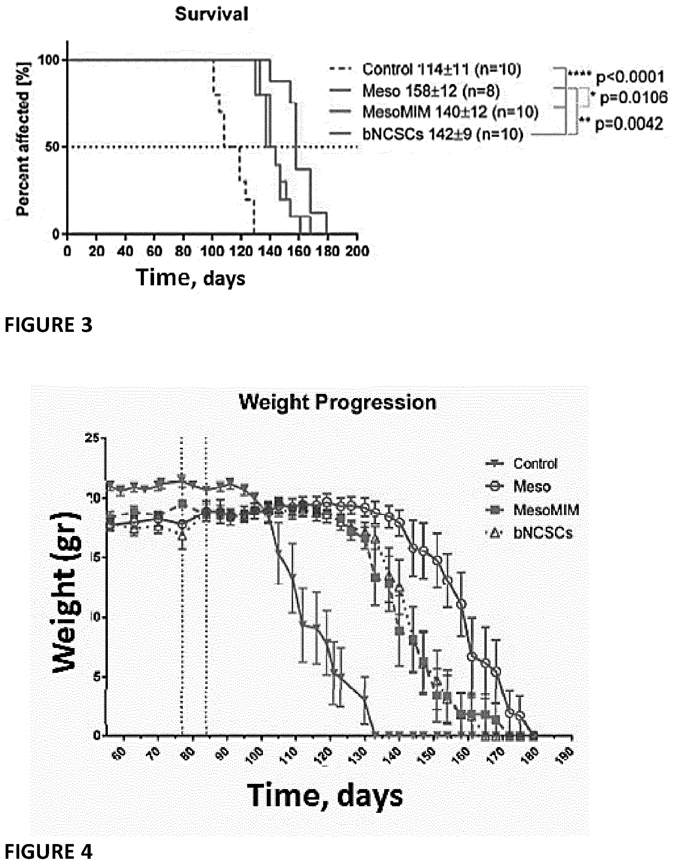

Fig. 3 shows that treatment with empty mesoporous silica particles or mimetic loaded mesoporous particles extends the survival of mutant SOD1G93A mouse model of ALS. Ages of disease end stage (determined as the time when the animal could not right itself within 20 seconds when placed on its side) of SOD1G93A and SOD1G93A littermates injected with mesoporous particles loaded with mimetics (MesoMIM,) (0.1 µg/ul particles), or with empty particles (Meso). (0.1 µg/ul particles) Mean ages ± S.D. is provided.
Fig. 4 shows that treatment with empty mesoporous silica particles or mimetic loaded mesoporous particles or bNCSCs (Stem Cells) extends weight progression of mutant SOD1G93A mouse model of ALS. SOD1G93A littermates injected with mesoporous particles loaded with mimetics (MesoMIM,) (0.1ug/µl particles), or with empty particles (Meso) (0.1ug/µl particles) or with bNSCs (~13,000cells/µl). Mean ages ± S.D is provided.

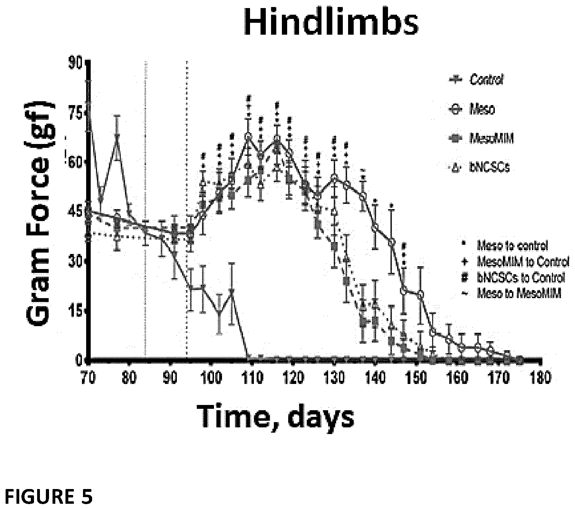

Fig. 5 shows a graph of averaged hindlimb grip strength for SOD1G93A injected with empty mesoporous particles or injected with mesoporous particles loaded with mimetics, or with bNCSCs cells at indicated age. The results are the means ± S.D.

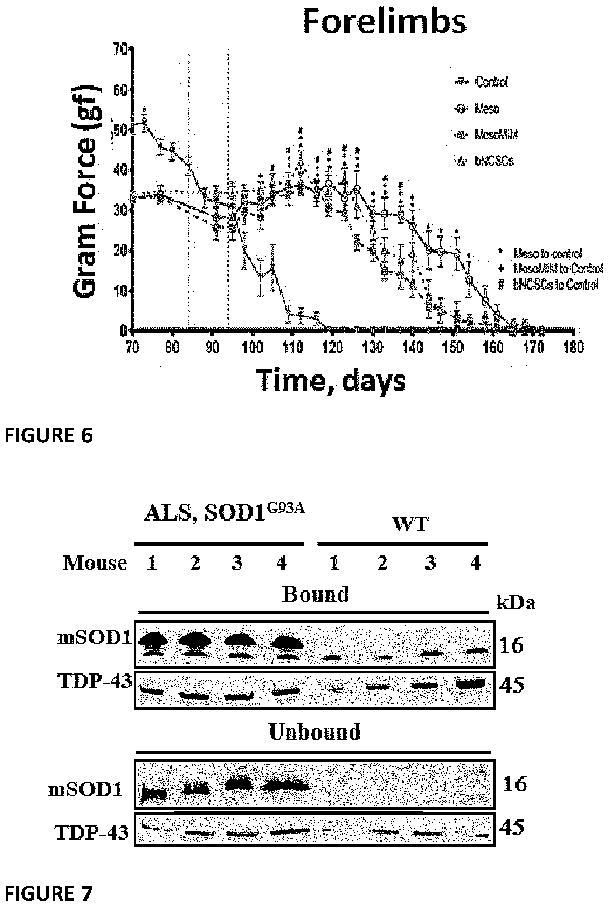

Fig. 6 shows a graph of averaged forelimb grip strength for SOD1G93A injected with empty mesoporous particles or injected with mesoporous particles loaded with mimetics, or with bNCSCs cells at indicated age. The results are the means ± S.D.
Fig. 7 shows immunoblot for mSOD-1 and TDP-43 of particles unbound and bound fractions obtained following incubation of the porous silica particles with spinal cord extracts obtained from Tg(SOD1^{∗}G93A) 1Gur/J mice (4 mice) and WT blac6 mice (4 mice), followed by separation of particles bound and unbound fractions.

### Detailed description of various embodiments of the invention

### Definitions and measurement methods

As used herein, the term "disease" is intended to include disorder, condition or any equivalent thereof.

The term "loading" means that the bioactive compound may be loaded into and/or onto the particle, without being fixedly bound to the particle.

As used herein, the term "substantially" or "about" refers to a deviation of a value around the number mentioned, e.g. substantially or about 5 means that the value may be between 4 and 6.

As used herein, the term "coating" refers to coverage of a surface, which may include blockage of the pores or not.

The term "extended release" means any release of a bioactive compound that is not immediate, i.e. not a release of at least 80% of the compound within 30 minutes.

As used herein, the term "patient" refers to a mammal, for example, a human.

As used herein, the term "at least a portion" means that at least 50w/w%, or at least 75w/w%, or at least 90 w/w% of all empty particles.

In the context of the present specification, the term "therapy" also includes "prophylaxis" unless there are specific indications to the contrary. The term "therapeutic" and "therapeutically" should be construed accordingly. The term "therapy" within the context of the present invention further encompasses to administer an effective amount of a compound of the present invention, to mitigate either a pre-existing disease state, acute or chronic, or a recurring condition. This definition also encompasses prophylactic therapies for prevention of recurring conditions and continued therapy for chronic diseases.

As used herein, the term "optional" or "optionally" means that the subsequently described event or circumstance may but need not occur, and that the description includes instances where the event or circumstance occurs and instances where it does not.

As used herein, the term "mimetics" refers to a small protein-like chain designed to mimetic a peptide. They typically arise either from modification of an existing peptide, or by designing similar systems that mimetic peptides, such as peptoids and β-peptides. Irrespective of the approach, the altered chemical structure is designed to advantageously adjust the molecular properties such as, stability or biological activity. This can have a role in the development of drug-like compounds from existing peptides.

As used herein, the term "postponement" refers to a delay of onset of a disease.

As used herein, the term "cervical and/or spinal fluid" refers to cerebrospinal fluid, brain or spinal cord parenchyma.

As used herein, the term "neural cells" or "neuronal cells" means all cells in the central nervous system (CNS), such as neurons and glia, such as astrocytes, oligodendrocytes and microglia.

Mercury (Hg) intrusion is a standard method for measuring porosity. In this method mercury is pushed into pores under applied pressure. However, there is a lower pore size limit of around 3.2 nm, below which, the mercury cannot penetrate. For porous materials with pore sizes in the mesoscale range of 1 to 50 nm, nitrogen (N₂) sorption is commonly used to estimate pore size and pore volumes. The nitrogen sorption technique measures the available surface area of the porous materials. An empirical model is used to calculate the pore volume and pore size. The BET (Brunauer-Emmett-Teller) and BJH (Barrett, Joyner and Halenda) models are used to calculate porosity for the pores of silica particles.

Scanning electron microscope (SEM) can be used to provide images of the porous particles. The diameter of the particles can be determined using SEM.

The empty porous particles may be manufactured by cooperative self-assembly of silica species and organic templates, such as cationic surfactants, such as alkyltrimethylammonium templates with varying carbon chain lengths, and counterions, such as cetyltrimethylammonium chloride (CTA+Cl- or CTAC) or cetyltrimethylammonium bromide (CTA+Br- or CTAB) or non-ionic species, such as diblock and triblock polymer species such as copolymers of Polyethylene Oxide and Polypropylene Oxide for example Pluronic 123 surfactant. The formation of mesoporous particles occurs following the hydrolysis and condensation of silica precursor, which can include alkylsilicates, such as tetraethylorthosilicate TEOS or teramethylorthosilicate TMOS in solution or sodium silicate solution. The mesoporous silica particle size can be controlled by adding suitable additive agents e.g. inorganic bases, alcohols including methanol, ethanol, propanol and organic solvents, such as acetone, which affect the hydrolysis and condensation of silica species. The pore size can be influenced by hydrothermal treatment of the reaction mixture, such as heating up to 100°C or even above, and also with the additional of swelling agents in the form of organic oils and liquids that expand the surfactant micelle template. After condensation of the silica matrix, the templating surfactant can be removed by calcination typically at temperatures from 500°C up to 650°C for several hours, which burns away the organic template resulting in a porous matrix of silica. The template may alternatively be removed by extraction and washing with suitable solvents, such as organic solvents or acidic of basic solutions.

The empty porous particles may be manufactured by a sol-gel method comprising of a condensation reaction of a silica precursor solution, such as sodium silicate or an aqueous suspension of silica nanoparticles as an emulsion, with a non-miscible organic solution, oil, or liquid polymer in which droplets are formed by for example stirring or spraying the solution followed by gelation of the silica by means of change in pH and or evaporation of the aqueous phase. The porosity of the empty particles here are formed either by exclusion due to the presence of the non-miscible secondary phase or by the jamming of the silica nanoparticles during evaporation. The empty particles may further be treated by heating to induce condensation of the silica matrix and washing to remove the non-miscible secondary phase. Furthermore, the empty particles may be treated by calcination to strengthen the silica matrix.

The empty porous particles may be manufactured as porous glass through a process of phase separation in borosilicate glasses (such as SiO₂-B₂O₃-Na₂O), followed by liquid extraction of one of the formed phases through the sol-gel process; or simply by sintering glass powder. During a thermal treatment, typically between 500°C and 760°C an interpenetration structure is generated, which results from a spinodal decomposition of the sodium-rich borate phase and the silica phase.

The porous empty particles may be manufactures using fumed process. In this method, fumed silica was produced by burning silicon tetrachloride in an oxygen-hydrogen flame producing microscopic droplets of molten silica, which fuse into amorphous silica particles in three-dimensional secondary particles which then agglomerate into tertiary particles. The resulting powder has an extremely low bulk density and high surface area.

The empty porous particles may have a diameter between 0.1 and 1000 µm, as measured by SEM, or between 0.1 and 500 µm, or between 0.1 and 250 µm, or between 0.1 and 100 µm, or between 0.2 and 50 µm, or between 0.3 and 25 µm, or between 0.3 and 20 µm, or between 0.3 and 12 µm, or between 0.3 and 6 µm. The empty particles may have nano- and/or mesopores.

The empty particles may have a pore size between 1 and 100 nm, or between 1 and 80 nm, or between 2 and 50 nm, or between 2 and 25 nm, or between 5 and 15 nm, or substantially 12 nm.

The empty particles may have a pore volume between 0.1 and 3 cm³/g, or between 0.2 and 2 cm³/g, or between 0.5 and 1.5 cm³/g, or between 0.7 and 1.2 cm³/g, or between 0.5 and 1.0 cm³/g, or substantially 8.5 cm³/g.

The empty particles may have a diameter between 0.1 and 1000 µm, a pore size between 1 and 100 nm, a pore volume between 0.1 and 3 cm³/g and a surface area between 40 and 1500 m²/g. The empty particles may have a pore size between 0.3 and 20 nm, a pore volume between 0.5 and 1.5 cm³/g and a surface area between 50 and 800 m²/g. The empty particles as used in the description may have any combination of the intervals mentioned above.

The empty particles may have different shapes. The shapes of the empty particles can be controlled by the process and may be spheres, pseudo-spheres, cylinders, gyroids, rods, fibres, core-shell shape of empty particles, or mixtures thereof. The empty particles may be substantially spherical or pseudo-spheres.

The empty porous particles may be porous silica particles. The silica may be any silica. The silica may be biodegradable and/or dissolvable. Examples of silica is amorphous silica or synthetic amorphous silica.

The empty porous particles may be manufactured by a process comprising or consisting of the steps of providing the silica particles, preferably by manufacturing by a sol-gel method or by a spray drying method,
-separating the empty particles. Separation may be done by air classifier, or cyclonic separation, or elutriation, or sedimentation and/or sieving using one or more sieves.

An incubator liquid may be loaded in or on all or at least a portion of the empty particles. Examples of such liquids may be any buffered liquid having a physiological pH of about 6.8, adapted for administration to a mammal, such as a human. Administration may be by injection.

Additional compounds, such as one or more mimetics or additional agents, or any combination thereof, may be loaded in or on all or at least a portion of the empty particles without being fixedly bound to the surface of the particles. Fixedly bound means that the agent or mimetic is permanently attached to the surface of the particles.

Examples of other mimetics may be, Gliafin and/or Cintrofin.

Examples of one or more additional agents may be growth factors, pH regulators, stabilizers, antibiotics, anti-inflammatory drugs and/or immune-suppressors.

The empty particles may be loaded using different techniques, such as solvent evaporation, impregnation, spray-drying, melting, supercritical CO₂ loading or freeze-drying, and the like. Solvent evaporation involves combining a concentrated solution of the bioactive compound with the silica particles, then removing the solvent and/or drying the sample prior to further processing.

The loading of the porous silica particles may be about 5 w/w% or more, or about 15, 20, 25%, 30, 40, 50, 75, 80, 85, 90 w/w%.

The empty porous particles may be biotinylated. The empty porous particles may be chemically modified by having a surface chemistry of terminal hydroxyl groups -OH, or having a hydrophobic surface chemistry, or having chemical functionalities including -COOH, -NH₃, OCH₃, -OCH₂CH₃, or mixtures thereof. A mixture of particles having different chemical modification may also be used.

Surface modification may be done prior to loading. Surface modification may be a chemical modification, such as etching. Etching may be done by boiling the silica particles in a base, and then in an acid to form -S-OH bonds on the outer surface of the particle. Examples of suitable bases may be a solution of KOH, a solution of NaOH or ammonia solution. Examples of suitable acids may be HNO₃, HCI and H₂SO₄.

The porous particles may be formulated into a pharmaceutical composition adapted for administration of the particles to a mammal, such as a human. The composition may comprise or consist of a plurality of porous particles together with a solvent, such as water or a biologically acceptable liquid adapted for administration to a mammal. The physiological pH is about 6.8 (physiological pH). The composition may further comprise or consist of pharmaceutical acceptable additives. Conventional procedures for the selection and preparation of suitable pharmaceutical compositions are described in, for example, "Pharmaceuticals - The Science of Dosage Form Designs", M. E. Aulton, Churchill Livingstone, 1988.

The pharmaceutical composition can be used for controlled or extended release of one or more mimetics or additional agents, or any combination thereof, loaded in or onto the porous particles. An extended release means a release of the loaded ingredient from the particles by less than 50% within 30 minutes from the start of a standard dissolution test, such as FDA Paddle Method (USP apparatus 2).

### Medical use.

As shown in the results, 10-week-old mice on B6/SJC background (B6SJL-TgN-SOD1-G93A-1Gur) were subjected to double or triple intracervical laminectomy surgery injection (2 µl) on the left side of the cervical spinal cord (C3-C4) after. Injection of empty nanoparticles or nanoparticles containing mimetics significantly delayed the disease course of SOD1 mutant mice.

The empty porous particles having a diameter between 0.1 and 1000 µm, as measured by SEM, or the pharmaceutical composition as defined above, may be used in treatment, prevention and/or postponement of degeneration of neurons and glia.

The empty porous particles or the pharmaceutical composition may be administered by injection in the spinal and/or cervical fluid. The composition can be injected anywhere in the spinal fluid or cervical fluid. The empty particles may be administered by injection in the cerebrospinal fluid (CSF), or in the brain or spinal cord parenchyma. The composition may be administered by injection in the spinal fluid between the third and fourth vertebrae.

The empty porous particles or the pharmaceutical composition as defined above, may be used in prevention and/or postponement of neurodegenerative diseases (NDDs), selected from the group comprising or consisting of Alzheimer's disease (AD), Parkinson's disease (PD), amyotrophic lateral sclerosis (ALS), Huntington's disease (HD), dementia with Lewy bodies (DLB), progressive supranuclear palsy (PSP), multiple system atrophy (MSA), corticobasal degeneration (CBD), frontotemporal dementia (FTD), spinocerebellar ataxia (SCA) disorders, and spinal muscular atrophy, or other NDDs.

The empty porous particles or the pharmaceutical composition as defined above, may be used in sequestering SOD1, TDP-43, amyloid-beta, alpha-synuclein, tau, ELAVL4, FUS and other biomolecules that affect degeneration of neurons and glia.

The empty porous particles or the pharmaceutical composition as defined above, may be used in sequestering SOD1, TDP-43, amyloid-beta, alpha-synuclein, tau, ELAVL4, FUS and other biomolecules that contribute to progression of neurodegenerative diseases (NDDs), selected from the group comprising or consisting of Alzheimer's disease (AD), Parkinson's disease (PD), amyotrophic lateral sclerosis (ALS), Huntington's disease (HD), dementia with Lewy bodies (DLB), progressive supranuclear palsy (PSP), multiple system atrophy (MSA), corticobasal degeneration (CBD), frontotemporal dementia (FTD), spinocerebellar ataxia (SCA) disorders, and spinal muscular atrophy and other NDDs

The empty porous particles or the pharmaceutical composition as defined above, may be used in sequestering SOD1, TDP-43, amyloid-beta, alpha-synuclein, tau, ELAVL4, FUS and other biomolecules that contribute to progression of neurodegenerative diseases (NDDs) before, during or after treatment of a mammal with porous particles at least partially loaded with trophic factors, such as peptide mimetics of Glial cell-derived neurotrophic factor (GDNF) and/or Ciliary neurotrophic factor (CNTF) and/or Stem Cells.

The empty porous particles or the pharmaceutical composition as defined above, may be used sequestering SOD1, TDP-43, amyloid-beta, alpha-synuclein, tau, ELAVL4, FUS and other biomolecules that contribute to progression of neurodegenerative diseases (NDDs) before, during or after treatment of a mammal with Stem Cells.

The empty porous particles may be used in prevention and/or postponement of NDDs in a mammal, whereby the empty particles are injected before, during or after treatment of the mammal with the particles loaded with trophic factors selected from peptide mimetics of Glial cell-derived neurotrophic factor (GDNF) and/or Ciliary neurotrophic factor (CNTF) and/or Stem Cells. The empty particles may be injected before said treatment. A mammal may be first treated by injection of empty particles and subsequently (within 1 to 24 hours) be treated by injection of particles that are at least partially loaded with trophic factors selected from peptide mimetics of Glial cell-derived neurotrophic factor (GDNF) and/or Ciliary neurotrophic factor (CNTF) and/or Stem Cells. Alternatively, a mammal may be simultaneously treated by injection of empty particles and by injection of particles that are at least partially loaded with trophic factors selected from peptide mimetics of Glial cell-derived neurotrophic factor (GDNF) and/or Ciliary neurotrophic factor (CNTF) and/or Stem Cells. Or, a mammal may be first treated by injection of particles that are at least partially loaded with trophic factors selected from peptide mimetics of Glial cell-derived neurotrophic factor (GDNF) and/or Ciliary neurotrophic factor (CNTF) and/or Stem Cells and subsequently (within 1 to 24 hours) be treated by injection of empty particles. The particles may be administered by injection in the cerebrospinal fluid (CSF), or in the brain or spinal cord parenchyma.

A dosage regime for administration of the pharmaceutical composition may be administration by injection in the spinal fluid at least once every month, or at least once every two months, or at least once every three weeks, or once every two weeks, or once every week.

### Diagnosis of a neurodegenerative diseases

The empty porous particles may be used in diagnosis of a neurodegenerative diseases (NDDs), selected from the group comprising or consisting of Alzheimer's disease (AD), Parkinson's disease (PD), amyotrophic lateral sclerosis (ALS), Huntington's disease (HD), dementia with Lewy bodies (DLB), progressive supranuclear palsy (PSP), multiple system atrophy (MSA), corticobasal degeneration (CBD), frontotemporal dementia (FTD), spinocerebellar ataxia (SCA) disorders and spinal muscular atrophy, or other NDDs.

The empty porous particles may be used in a method of
identifying biomarkers for diagnosis and/or treatment of NDDs, such as ALS comprising the steps of:
a) administering the empty porous particles having a diameter between 0.1 and 1000 µm, as measured by SEM, as defined above into the cerebrospinal fluid (CSF);
b) retrieving a portion of said porous particles after a period of time, such as 1 to 36 hours, from the CSF;
c) determining biomolecules that have been loaded into the porous particles during their presence in the CSF;
d) establishing if the compounds can be used as biomarkers.

The period of time in step b) may be 1 to 24 hours or 1 to 72 hours.

All or at least a portion of the empty particles may be loaded with an incubation liquid as mentioned above.

Administration means in vivo administration as well as in vitro addition of empty particles to CSF retrieved from a mammal.

Thus, step a) may be adding the empty porous particles as defined herein, to the cerebrospinal fluid (CSF) retrieved from a mammal in vitro.

The identified biomarkers may be used in for diagnosis, prevention, treatment and/or postponement of NDDs, such as ALS; or NDDs selected from the group comprising or consisting of Alzheimer's disease (AD), Parkinson's disease (PD), Huntington's disease (HD), dementia with Lewy bodies (DLB), progressive supranuclear palsy (PSP), multiple system atrophy (MSA), corticobasal degeneration (CBD), frontotemporal dementia (FTD), spinocerebellar ataxia (SCA) disorders and spinal muscular atrophy.

### Experimental

### Synthesis of mesoporous silica particles.

Pluronic 123 (triblock co-polymer, EO₂₀PO₇₀EO₂₀, Sigma-Aldrich) (4 g) as a templating agent and, 1,3,5-trimethylbenzene (TMB) (Mesitylene, Sigma-Aldrich) (3.3 g) as swelling agent were dissolved in 127 ml distilled H₂O and 20 ml hydrochloric acid (HCl,37%, Sigma-Aldrich) while stirring at room temperature (RT) for 3 days. The solution was preheated to 40°C before adding 9.14 ml TEOS (Tetraethyl orthosilicate, Sigma-Aldrich). The mixture was stirred for another 10 mins at the speed of 500 rpm and then kept at 40°C for 24 hours, then hydrothermally treated in the oven at 100°C for another 24 hours. Finally, the mixture was filtered, washed and dried at room temperature. The product was calcined to remove the surfactant template and swelling agent. The calcination was conducted by heating to 600°C with a heating rate of 1.5°C/min and kept at 600°C for 6 hours followed by cooling to ambient conditions. The resulting product is a white powder comprising of nanoporous silica particles.

### Synthesis of Peptide Mimetics

The peptides Cintrofin and Gliafin, derived from the ciliary neurotrophic factor (148-DGGLFEKKLWGLKV-161; UniProtKB entry no. P26441) and glial cell line-derived neurotrophic factor (153- ETMYDKILKNLSRSR-167; UniProtKB entry no. Q07731), respectively,
were synthesized using the solid-phase Fmoc protection strategy, and purity was estimated to be at least 80% by high performance liquid chromatography. All of the peptides were synthesized by Schafer-N AS (Copenhagen, Denmark, http://www.schafer-n.com) as dendrimers composed of four monomers coupled to a lysine backbone. During the synthesis of biotinylated peptides, only N-terminal amino acids were biotin labeled amino acids. Thus, each tetrameric dendrimer contained four biotin residues.

Loading Peptide Mimetics Gliafin and Cintrofin.

The peptide mimetics were loaded via impregnation in water. Mesoporous silica particles (50 mg) were added to 0.5 ml of Gliafin in a water solution at a concentration of 8.87 mg/ml and stirred at 4°C for 16 hours. Mesoporous silica particles (25 mg) were added to a 0.4-ml Cintrofin water solution at a concentration of 4.4 mg/ml and stirred at 4°C for 16 hours. The water in both solutions was evaporated under atmospheric conditions. The loading amount of peptide was determined by thermogravimetric analysis (PerkinElmer). Scanning was performed from 20°C to 900°C at a heating rate of 20°C/minute. The plug-in gas atmosphere was dry air (flow rate, 20ml/minute). The sample weights varied from 5 to 10 mg. Loading efficiencies of mesopores were 8.3 and 11.8 wt% for Cintrofin and Gliafin, respectively.

### Preparation of particle solutions

The solutions were prepared on the day of the start of the experiments.

### Dispersion of mesoporous particle solution

A 1 µg/µl concentration was made by mixing an equal volume of solvent or injection solution to dry powder of mesoporous silica particles. The mixture was mixed thoroughly. 100 µl of the final solution was put in a sterile tube for injection.

### Mimetic solution

1 µg/µl of each concentrated Gliafin, Cintrofin loaded mesoporous silica particle solution was prepared as above. 100 µl from each tube was added to a sterile tube and 900 µl of solvent was added. The mixture was mixed thoroughly. 100 µl of the final solution was put in a sterile tube for injection. Final concentration of each mimetic was 0.1 µg/µl.

### Mice injections and survival

At 79 days (about 10 week) B6/SJL background (B6SJL-TgN-SOD1-G93A-1Gur) mice were anesthetized with 3% isoflurane at the beginning of the procedure and decreasing over the time till reaching 0.8% at a flow rate of 500-480ml/min. A partial laminectomy was made over the left cervical spinal cord, and double or triple intracervical injection was made into the ventral horn on the left side of spinal cord segments C3-C4. A 2 µl intracervical injection was performed in the three groups; empty porous silica particles(0.1 µg/µl), mimetic group (Gliafin, Cintrofin loaded mesoporous silica particles, 0.1 µg/µl), bNCSCs (boundary cap neural crest stem cells, about 13,000 cells/µl). A 10µl Hamilton syringe with a point style AS needle was attached to a micro syringe pump controller (Micro 4, WPI), set at an infusion pace of 4 µl/min.

For survival experiments, SOD1G93A mice injected with mimetics or boundary cap neural crest stem cells (bNCSCs) were always compared with their littermates injected with empty particles. Time of disease onset was retrospectively determined as the time when mice reached peak body weight. Early disease was defined at the time when denervation-induced muscle atrophy had produced a 10% loss of maximal weight. End-stage was determined by paralysis so severe that the animal could not right itself within 20 seconds when placed on its side, an endpoint frequently used for SOD1 mutant mice and one that was consistent with the requirements of the Animal Care and Use Committee of Ben-Gurion University of the Negev.

Grip strength test was performed with Chatillon force measurement device, Ametek, measurement of each member of the groups included two measurements (forelimb pulling and total limb pulling). Measurements were performed twice a week beginning two weeks before the surgery.

### Cell death analysis

Cells are incubated with the indicated mimetics in the appropriate serum-free growth medium at 37°C, harvested, washed twice with PBS and cell death is analyzed by propidium iodide (PI) staining and flow cytometry.

### Determination of neurite outgrowth intersections between neurites and survival

Stereological estimation of neurite length to evaluate neurite outgrowth in cells in culture was carried out as described previously (Rønn LC, Ralets I, Hartz BP, Bech M, Berezin A, Berezin V, Møller A, Bock E. Asimple procedure for quantification of neurite outgrowth based on stereological principles. J Neurosci Methods. 2000 Jul 31;100(1-2):25-32). The total neurite length per cell was estimated by counting the number of intersections between neurites and test lines of an unbiased counting frame superimposed on images of cell cultures obtained by conventional computer-assisted microscopy. The absolute length (L) of neurites per cell was subsequently estimated from the number of neurite intersections (I) per cell by means of the equation L=(πd/2)l describing the relationship between the number of neurite intersections and the vertical distance d between the test lines used.

In vivo experiment is done using a mouse model for ALS (mouse heterozygous for the mutant SOD1 transgene). In addition, the effect of the selected mimetics on neurite outgrowth, MN density and survival is tested.

### Treatment with empty nanoparticles, mimetics or bNCSCs extends the survival of mutant SOD1G93A mice

It was examined how injection of mesoporous silica particles containing mimetics or direct injection of bNCSCs will affect disease course in SOD1 mutant mice. To do this, the B6SJL-TgN-SOD1-^{G93A}-1Gur (SOD1G93A) mice, heterozygous for the mutant SOD1 transgene, was used. These mice develop progressive motor neuron disease and have a median survival of 128.9± 9.1 days (Gurney et al., 1994). 79 days old mice were injected with nanoparticles containing mimetics (n=10) or with empty nanoparticles (n=10) (Fig. 1). A simple and objective measure of disease onset and early disease progression was applied by determining the initiation of weight loss, which reflects denervation-induced muscle atrophy.

Surprisingly, while timing of disease end stage was extended by injection of mesoporous silica particles loaded with mimetics (140±5 days), the injection of empty nanoparticles had the strongest survival effect (158±4 days) (Fig. 1), demonstrating that these porous particles improved the disease course.

In addition, the ability of boundary cap neural crest stem cells (bNCSCs) to affect disease course was tested. Injection of bNCSCs cells to the SOD1G93A mice extended survival (142±3.5 days) (Fig. 2), at a similar extent as the nanoparticles loaded with mimetics.

This strong effect of the empty porous particles can be clearly observed by the results from measurements of grip strength. The empty porous particles group showed a significant (p=≤ 0.01, multi t-test) enhancement in the pulling strength from hindlimbs (28.8 g) (Fig. 3) and forelimbs (49.61 g) (Fig. 4) at 137 days compared to the group of mimetics or the bNCSC group. Overall, the empty porous particles group showed a significant improvement (p=≤ 0.01, multi t-test) in the forelimbs throughout the development of the disease at 133 days (29 g), 137 days (28.8 g) and 151 days (19.16) compared to the other groups (mimetics or bNCSCs) (Fig. 4). These results suggest that mice injected with empty particles retain their muscular strength mainly in the forelimbs. This can be explained by the proximity of the forelimb innervating motor neurons to the injection site (C3-C4).

### Mesoporous silica particles sequester proteins/peptides including proteins considered as hallmarks for NDDs.

Spinal cord extracts were obtained from four 12 weeks old mice, mutant SOD1-^{G93A}-B6SJL-Tg(SOD1^{∗}G93A) 1Gur/J mice (4 mice) and WT blac6 mice (4 mice) by homogenization in a buffer (210 mM mannitol, 70 mM sucrose, 10 mM Tris, pH 7.4 and a protease inhibitors) , centrifuged (12,000 g, 10 min). The mitochondria-free supernatants (50 µg protein) from SOD1^{∗}G93A and wt mice were incubated for 5h with 10 µg MSPs particles (37°C 300 RPM), followed by centrifugation (12,000 g, 10 min). The obtained supernatants **(unbound fraction)** were subjected to immunoblotting. The obtained mesoporous silica particles pellets were washed with 10 mM Tris, 50 mM NaCl, pH 7.4 and the resulting pellet was resuspended in a 50 µl of lysis buffer (100 mM Tris-HCI, pH 8.0, 5 mM DTT 4% SDS and a protease inhibitor cocktail), incubated for 15 min at 50°C, 300 RPM and centrifuged (20 min, 12,000 g) to obtain the **bound fraction**. Unbound and bound fractions were subjected to SDS-PAGE, and immunoblotting using the indicated antibodies. (Fig 5).

The results demonstrate the porous particles high capacity to absorb proteins as mutant SOD1, and TDP-43. We expect the particles absorbed other proteins, although their entity has not been identified yet. Using proteomics and bioinformatics analysis, we expect to identify the key proteins that are absorbed by MSPs, thereby, eliminating their toxic effects associated with the pathology conditions of ALS and other NDDs. These proteins/factors can be used as biomarker for early detection and/or as new drug targets.

Development and remodeling of neuronal extensions (neurite outgrowth) in the presence of empty particles (Ivert et al, Ivert P, Otterbeck A, Panchenko M, HoeberJ, Vasylovska S, Zhou C, Garcia Bennett A, Kozlova EN. The Effect of Mesoporous Silica Particles on Stem Cell Differentiation. J Stem Cell Res Ther. 2017;2(3):73-78. DOI: 10.15406/jsrt.2017.02.00063) in cell culture was determined by tracing neurites and their branches using the stereological procedure as previously described (Ronn et al., A simple procedure for quantification of neurite outgrowth based on stereological principles. J Neurosci Methods. 2000 Jul 31;100(1-2):25-32.).

The present invention is not limited to the embodiments disclosed but may be varied and modified within the scope of the following claims.

## Claims

1. Empty mesoporous silica particles having a diameter between 0.1 and 1000 µm, for use in prevention and/or postponement of degeneration of neurodegenerative diseases (NDDs) in a mammal, wherein co-transplantation of stem cells is disclaimed, and the empty particles have no surface bound molecules or agents, and wherein the neurodegenerative diseases is selected from the group consisting of Alzheimer's disease (AD), Parkinson's disease (PD), amyotrophic lateral sclerosis (ALS), Huntington's disease (HD), dementia with Lewy bodies (DLB), progressive supranuclear palsy (PSP), multiple system atrophy (MSA), corticobasal degeneration (CBD), frontotemporal dementia (FTD), spinocerebellar ataxia (SCA) disorders and spinal muscular atrophy.

2. Empty porous particles for use according to claim 1, for use in prevention and/or postponement of degeneration of neurons and glia in a mammal.

3. Empty porous particles for use according to any one of the preceding claims, for use in prevention and/or postponement of NDDs in a mammal, whereby the empty particles are administered before, during or after treatment of the mammal with, all or at least a portion of the particles loaded with trophic factors selected from peptide mimetics of Glial cell-derived neurotrophic factor (GDNF) and/or Ciliary neurotrophic factor (CNTF) and/or Stem Cells.

4. Empty porous particles for use according to any one of the preceding claims, wherein the empty particles are mesoporous silica particles having a pore size between 1 and 100 nm, a pore volume between 0.1 and 3 cm³/g and a surface area between 40 and 1500 m²/g.

5. Empty porous particles for use according to any one of the preceding claims, wherein the empty particles have a pore size between 0.3 and 20 nm, a pore volume between 0.5 and 1.5 cm³/g and a surface area between 50 and 800 m²/g.

6. Empty porous particles for use according to any one of the preceding claims, wherein the empty particles have a diameter between 0.1 and 500 µm, or between 0.1 and 250 µm, or between 0.1 and 100 µm, or between 0.2 and 50 µm, or between 0.3 and 25 µm, or between 0.3 and 20 µηι, or between 0.3 and 12 µm, or between 0.3 and 6 µm.

7. Empty porous particles for use according to any one of the preceding claims, wherein the empty particles are administered by injection in the cerebrospinal fluid (CSF), brain or spinal cord parenchyma.

8. Empty porous particles for use according to any one of the preceding claims, wherein an incubator liquid is loaded in or on all or at least a portion of the empty particles, and the incubator liquid is a buffered solution having a physiological pH suitable for administration by injection.

9. Empty porous particles for use according to any one of the preceding claims, wherein the empty particles are biotinylated.

## Patentansprüche

1. Leere mesoporöse Siliciumdioxidpartikel mit einem Durchmesser zwischen 0,1 und 1000 µm, zur Verwendung in der Prävention und/oder Aufschiebung der Degeneration von neurodegenerativen Erkrankungen (NDDs) in einem Säugetier, wobei die Co-Transplantation von Stammzellen abgelehnt wird, und die leeren Partikel keine oberflächengebundenen Moleküle oder Mittel aufweisen, und wobei die neurodegenerativen Erkrankungen ausgewählt sind aus der Gruppe bestehend aus Alzheimer-Krankheit (AD), Parkinson-Krankheit (PD), amyotropher Lateralsklerose (ALS), Huntington-Krankheit (HD), Lewy-Körper-Demenz (DLB), der progressiven supranukleären Paralyse (PSP), der multiplen Systematrophie (MSA), der kortikobasalen Degeneration (CBD), der frontotemporalen Demenz (FTD), der spinozerebellären Ataxie (SCA) und der spinalen Muskelatrophie.

2. Leere poröse Partikel zur Verwendung nach Anspruch 1, zur Verwendung in der Prävention und/oder Aufschiebung von Neuronen und Glia in einem Säugetier.

3. Leere poröse Partikel zur Verwendung nach einem der vorstehenden Ansprüche, zur Verwendung in der Prävention und/oder Aufschiebung von NDDs in einem Säugetier, wobei die leeren Partikel vor, während oder nach der Behandlung des Säugetiers mit allen oder mindestens einem Teil der mit trophen Faktoren beladenen Partikel verabreicht werden, die aus Peptidmimetika des von Gliazellen abgeleiteten neurotrophen Faktors (GDNF) und/oder des ziliären neurotrophen Faktors (CNTF) und/oder Stammzellen ausgewählt sind.

4. Leere poröse Partikel zur Verwendung nach einem der vorstehenden Ansprüche, wobei die leeren Partikel mesoporöse Siliciumdioxidpartikel mit einer Porengröße zwischen 1 und 100 nm, einem Porenvolumen zwischen 0,1 und 3 cm³/g und einer Oberfläche zwischen 40 und 1500 m²/g sind.

5. Leere poröse Partikel zur Verwendung nach einem der vorstehenden Ansprüche, wobei die leeren Partikel eine Porengröße zwischen 0,3 und 20 nm, ein Porenvolumen zwischen 0,5 und 1,5 cm³/g und eine Oberfläche zwischen 50 und 800 m²/g aufweisen.

6. Leere poröse Partikel zur Verwendung nach einem der vorstehenden Ansprüche, wobei die leeren Partikel einen Durchmesser zwischen 0,1 und 500 µm oder zwischen 0,1 und 250 µm oder zwischen 0,1 und 100 µm oder zwischen 0,2 und 50 µm oder zwischen 0,3 und 25 µm oder zwischen 0,3 und 20 µm oder zwischen 0,3 und 12 µm oder zwischen 0,3 und 6 µm aufweisen.

7. Leere poröse Partikel zur Verwendung nach einem der vorstehenden Ansprüche, wobei die leeren Partikel durch Injektion in die Gehirn- und Rückenmarksflüssigkeit (CSF), das Gehirn oder das Rückenmarkparenchym verabreicht werden.

8. Leere poröse Partikel zur Verwendung nach einem der vorstehenden Ansprüche, wobei eine Inkubatorflüssigkeit in oder auf alle oder mindestens einen Teil der leeren Partikel geladen ist, und die Inkubatorflüssigkeit eine Pufferlösung ist, die einen physiologischen pH-Wert aufweist, der zur Verabreichung durch Injektion geeignet ist.

9. Leere poröse Partikel zur Verwendung nach einem der vorstehenden Ansprüche, wobei die leeren Partikel biotinyliert sind.

## Revendications

1. Particules de silice mésoporeuses vides ayant un diamètre compris entre 0,1 et 1000 µηι, destinées à être utilisées pour la prévention et/ou le report de la dégénérescence de maladies neurodégénératives (MND) chez un mammifère, dans lesquelles la co-transplantation de cellules souches est exclue, et les particules vides n'ont pas de molécules ou agents liés à la surface, et dans lesquelles les maladies neurodégénératives est choisie dans le groupe constitué par la maladie d'Alzheimer (MA), la maladie de Parkinson (MP), la sclérose latérale amyotrophique (SLA), la maladie de Huntington (MH), la démence à corps de Lewy (DCL), la paralysie supranucléaire progressive (PSP), l'atrophie multisystématisée (AMS), la dégénérescence corticobasale (DCB), la démence frontotemporale (DFT), les troubles d'ataxie spinocérébelleuse (ASC) et l'amyotrophie spinale.

2. Particules poreuses vides destinées à être utilisées selon la revendication 1, destinées à être utilisées pour la prévention et/ou le report de la dégénérescence de neurones et de la glie chez un mammifère.

3. Particules poreuses vides destinées à être utilisées selon l'une quelconque des revendications précédentes, destinées à être utilisées pour la prévention et/ou le report de MND chez un mammifère, moyennant quoi les particules vides sont administrées avant, pendant ou après le traitement du mammifère avec, la totalité ou au moins une partie des particules chargées de facteurs trophiques choisis parmi des mimétiques peptidiques du facteur neurotrophique dérivé des cellules gliales (GDNF) et/ou du facteur neurotrophique ciliaire (CNTF) et/ou des cellules souches.

4. Particules poreuses vides destinées à être utilisées selon l'une quelconque des revendications précédentes, dans lesquelles les particules vides sont des particules de silice mésoporeuses ayant une taille des pores comprise entre 1 et 100 nm, un volume des pores compris entre 0,1 et 3 cm³/g et une superficie comprise entre 40 et 1 500 m²/g.

5. Particules poreuses vides destinées à être utilisées selon l'une quelconque des revendications précédentes, dans lesquelles les particules vides ont une taille des pores comprise entre 0,3 et 20 nm, un volume des pores compris entre 0,5 et 1,5 cm³/g et une superficie comprise entre 50 et 800 m²/g.

6. Particules poreuses vides destinées à être utilisées selon l'une quelconque des revendications précédentes, dans lesquelles les particules vides ont un diamètre compris entre 0,1 et 500 µηι, ou entre 0,1 et 250 µηι, ou entre 0,1 et 100 µηι, ou entre 0,2 et 50 µηι, ou entre 0,3 et 25 µηι, ou entre 0,3 et 20 µηι, ou entre 0,3 et 12 µηι, ou entre 0,3 et 6 µm.

7. Particules poreuses vides destinées à être utilisées selon l'une quelconque des revendications précédentes, dans lesquelles les particules vides sont administrées par injection dans le liquide céphalo-rachidien (LCR), le cerveau ou le parenchyme médullaire.

8. Particules poreuses vides destinées à être utilisées selon l'une quelconque des revendications précédentes, dans lesquelles un liquide incubateur est chargé dans ou sur la totalité ou au moins une partie des particules vides, et le liquide incubateur est une solution tamponnée ayant un pH physiologique appropriée pour une administration par injection.

9. Particules poreuses vides destinées à être utilisées selon l'une quelconque des revendications précédentes, dans lesquelles les particules vides sont biotinylées.
